# EUROPEAN PATENT APPLICATION

(11) **EP 4 389 105 A1**
(43) Date of publication of application: **26.06.2024**
(21) Application number: 22215088.0
(22) Date of filing: 20.12.2022
(51) Int. Cl.: A61K 6/62, A61K 6/76, A61K 6/77, A61K 6/887

(54) **DENTAL COMPOSITION COMPRISING A POLYMERIZATION SHRINKAGE MEDIATOR**

(71) Applicant: Dentsply DeTrey GmbH, 78467 Konstanz (DE)
(72) Inventor: RITTER, Helmut, 78467 Konstanz (DE); LU, Hui, Milford, 19963 (US); KLEE, Joachim E., 78467 Konstanz (DE); SPERBER, Rolf, 42329 Wuppertal (DE); HERD, Oliver, 45549 Sprockhövel (DE)
(74) Representative: Pichova, Vanda

(57) **Abstract**

The present invention relates to a dental composition comprising at least one polymerization shrinkage mediator. More particularly, the present invention relates to a dental composition comprising at least one polymerizable monomer, at least one polymerization initiator and at least one polymerization shrinkage mediator, wherein the at least one polymerization shrinkage mediator is selected from a group comprising at least one phenylacrylate. The at least one phenylacrylate is a substituted or unsubstituted phenylacrylate.

The present invention also releates to a dental composition comprising the phenylacrylate in a high purity. The dental composition is for production of a dental restoration material, a dental adhesive, a dental cement, a post and core material, an endo-crown material or a pontic in fixed partial dentures. The dental composition is for medical applications.

## Description

### FIELD OF THE INVENTION

The present invention relates to a dental composition comprising at least one polymerization shrinkage mediator.

### BACKGROUND OF THE INVENTION

Majority of the resin matrix for current dental composites and resin cements are cured via free radical polymerization. Well-preserved interfacial bonding between the composite and tooth is of vital importance to prevent the occurrences of compromised marginal integrity, microleakage, and recurrent caries. Rapid development of polymerization shrinkage and subsequent shrinkage induced stress have been major drawbacks in such systems and hence strategies to mediate the detrimental impacts of polymerization shrinkage stress could provide significant benefits toward product performance and longevity, and ultimately benefit the patients and their oral health.

EP 3566689 B1 of Shofu Inc. discloses a dental adhesive composition including chain transfer agent of alfa-alkyl styrene compound having self-adhesive property; it becomes possible to reduce the polymerization shrinkage stress.

EP 3610845 A1 of Stick Tech Oy discloses a dental composition used as a dental restoration material and an endo-crown material for pontic in fixed paral denture. The composition comprises alfa-methyl styrene derivatives to reduce the polymerization shrinkage stress.

Numerous efforts have been made in the past to reduce the polymerization stress. These include flexibilized networks, reducing the number of double bonds per molecule through the use of prepolymers (WO 98/36729, WO 01/08639), the application of stable radicals (EP 3052481) and the use of nanogels (US 111 10037). Both flexibilized networks and the reduction in the number of double bonds lead to a reduction in the mechanical properties. In principle, nanogels can fulfill the function of stress reduction, but they are very complex to produce and therefore expensive.

### BRIEF SUMMARY OF THE INVENTION

This invention discloses the solution of mediating polymerization shrinkage stress via mediators that can form hindered carbon-centered radicals. Such stable carbon-centered radicals, for example acrylates, have capabilities to couple reversibly with propagating radicals.

We hereby disclose a new class of novel phenylacrylates that are able to mediate polymerization, reduce both the magnitude and rate of polymerization shrinkage stress development, via hindered carbon centered radical. It is also assumed that in addition to the steric effect, electronic effect and stereoelectronic effect could also contribute to the overall mediation of the polymerization and subsequent polymerization shrinkage stress mitigation as described.

Such unique compounds are to be first designed, synthetized, analyzed and the optimized concentration to be identified to maximize the polymerization shrinkage stress mediating effect, while not sacrificing other key properties. DOE could be utilized, along with verification and biocompatibility and performance validity within the shelf life after which the verified compound will be incorporated into commercial dental composite/ resin formulations at the optimized concentration.

### BRIEF DESCRIPTION OF THE FIGURES

**Table 1** depicts dental compositions (resins, pastes) used in the present invention
**Table 2** depicts polymerization shrinkage stress and other characteristics of single resins
**Table 3** depicts polymerization shrinkage stress and other characteristics of single pastes
**Table 4** depicts characteristics used to define properties of the resins and pastes of the present invention and their units

### DETAILED DESCRIPTION

The present invention relates to a dental composition comprising at least one polymerization shrinkage mediator. More particularly, the present invention relates to a dental composition comprising at least one polymerizable monomer, at least one polymerization initiator and at least one polymerization shrinkage mediator.

The dental composition can further comprise at least one filler.

In the preferred embodiment of the present invention, the dental composition comprises a polymerization shrinkage mediator.

The dental compositions are preferably in their uncured form in the dental composition before its application and are cured once the composition is placed into its final position (such as in a dental to be restored). The curing may be induced by light, heat or by a combination of an initiator/activator and light, or other wave energy such as UV or ultrasonic activation. By curing, it is meant either polymerization or cross-linking or another method of curing.

The material of the at least one filler is typically a particulate filler material, preferably selected from the group comprising calcium alumo silicate glass, calcium alumo fluorosilicate glass, calcium alumo fluoroborosilicate glass, strontium alumo silicate glass, strontium alumo fluorosilicate glass, strontium alumo fluoroborosilicate glass, and barium alumo borosilicate glass, barium alumo silicate glass.

The dental composition comprises preferably 50 - 85 wt% of the at least one filler.

The at least one polymerization initiator is preferably at least one photo polymerization initiator selected from the group comprising Norrish Type I and Norrish Type II photo polymerization initiators, from the group comprising mono and diacylphosphine oxides, camphorquinone, camphorquinone/amine, camphor quinone/triphenylgermanium hydride (Ph3GeH) / diphenyl iodonium hexafluorophosphate, DKSI/DPI and dimethyl-DPI, preferably camphorquinone/arylamine.

The dental composition comprises preferably 0.2-3.0 wt% of the at least one polymerization initiator.

The at least one polymerization shrinkage mediator is selected from a group comprising at least one phenylacrylate. The at least one phenylacrylate is a substituted or unsubstituted phenylacrylate, preferably E2PA.

The dental composition comprises preferably 0.1 - 2.0 wt % of the at least one polymerization shrinkage mediator.

In one embodiment of the present invention, the at least one polymerizable monomer is selected from the group comprising an acrylate or a methacrylate.

The present invention also relates to the dental composition for a dental restoration material, a dental adhesive, a dental cement, a post and core material, and endo-crown material or for a pontic in fixed partial dentures. The present invention further relates to the dental composition for medical applications.

### EXAMPLES

**The following dental compositions are used in the present invention:**

**Table 1**

| **wt% E2PA** | **E2PA purity** | **Resin** | **Supplier** | **Relevant paste** | **wt% E2PA in the paste** |
|---|---|---|---|---|---|
| 0 | - | CMX Liq. | - | Ceram X Universal | 0 |
| 0.625 | 97% | HLU19-115R1 | Ambeed | HLU19-116P | 0.143 |
| 0.625 | 99% | HLU19-189R1 | ChemCollect | HLU19-195P | 0.143 |
| 1.00 | 99% | HLU19-194R | ChemCollect | - | - |
| 1.25 | 97% | HLU19-114R2 | Ambeed | - | - |
| 1.25 | 99% | HLU19-189R2 | ChemCollect | - | - |

### The following abbreviations are used in the present invention:

E2PA Ethyl-2-phenylacrylate
PSS Polymerization shrinkage stress
EBPADMA ethoxylated bisphenol A dimethacrylate
DKSI tertbutyl(dimethylsilyl)glyoxylate
DOE Design of Experiments (Statistical method)
DPI Diphenyleneiodonium
CQ Camphorquinone

### Production of CMX Liq.

The following components were mixed homogenously:
727.50 g of Ormosil, 6547.50 g of EBPADMA Urethane resin, 55.50 g of Dimethyl-DPI, 37.50g of Ralox BHT Foodgrade, 075 g of Lumilux blau, 45.0 g of 4-Hydroxy-2-Methoxybenzophenone, 11.25 g of camphorquinone , and 75 g of Ethyldimethylaminobenzoate.

### Production of single resins

To CMX Liq. were added Ethyl-2-phenylacrylate (E2PA) and stirred for one hour at room temperature.

**Table 2**

| The following table depicts various characteristics of the single resins described above, including PSS values. | | | | | | | | | | | | |
|---|---|---|---|---|---|---|---|---|---|---|---|---|
| **Resin** | **E2PA conc** | **E2PA purity** | **FS** | **FM** | **FT** | **PSS** | **PSS red control** | **Max PSS rate** | **Max PSS rate red control** | **ΔH** | **Exo Peak Scope** | **G'/G" crossover** |
| CMX Liq. | 0 | - | 108 | 2457 | 1.04 | **4.05** | - | **15.4** | - | 133 | 65.90 | 8.8 |
| HLU19-115R1 | 0.625 wt% | 97% | 104 | 2409 | | **2.88** | **29%** | **5.6** | **63%** | 132.8 | 8.24 | 16.3 |
| HLU19-189R1 | 0.625 wt% | 99% | 103 | 2362 | 1.56 | **1.96** | **52%** | **2.3** | **85%** | 130.8 | 2.61 | |
| HLU19-194R | 1.00 wt% | 99% | 103 | 2336 | 1.53 | **1.98** | **51%** | **2.2** | **86%** | 126.2 | 2.18 | |
| HLU19-114R2 | 1.25 wt% | 97% | 91 | 2054 | | **2.11** | **48%** | **2.3** | **85%** | 126.6 | 2.59 | 31.5 |
| HLU19-189R2 | 1.25 wt% | 99% | 89 | 1979 | 1.51 | **1.65** | **59%** | **1.7** | **89%** | 122.4 | 1.19 | |

It is clearly demonstrated, that the presence of E2PA reduces the PSS and that the higher level of purity of E2PA reduces the PSS whilst not sacrificing other key properties.

### Production of single pastes

To CMX Liq. were added Ethyl-2-phenylacrylate (E2PA) and stirred for one hour at room temperature. Then barium alumo silicate glass was added to the polymerizable liquid and stirred until a homogeneous paste was obtained. The mixture was then degassed at 200 mbar and 40°C for 15 minutes.

The following table depicts various characteristics of the single pastes described above, including PSS.

**Table 3**

| **Paste** | **Resin** | **Filler wt%** | **E2PA Conc in resin** | **Purity E2PA** | **E2PA Cone in paste** | **FS** | **FM** | **cs** | **FT** | **Resin PSS** | **Paste PSS** | **ISO 20'-VLC DoC** | **PSS red control paste** |
|---|---|---|---|---|---|---|---|---|---|---|---|---|---|
| Ceram X Universal (TPH Spectra ST) | CMX Liq. | 77.2 | 0 wt% | - | 0 | 128 | 8127 | 364 | 1.45 | **4.05** | **2.55** | 2.4 | - |
| HLU 19-116P | HU19-115R1 | 77.2 | 0.625 wt% | 97% | 0.143 | 132 | 7651 | 355 | 1.42 | **2.88** | **2.12** | 4.4 | **17%** |
| HLU 19-195P | HLU19 -189R1 | 77.2 | 0.625 wt% | 99% | 0.143 | 135 | 8053 | 358 | 1.71 | **1.96** | **1.92** | 4.7 | **25%** |

It is clearly demonstrated, that the presence of E2PA reduces the PSS and that the higher level of purity of E2PA reduces the PSS whilst not sacrificing other key properties.

All the above described characteristics and their units are summarized in the following table:

**Table 4**

| **Abbreviation/Term** | **Characteristic** | **Unit** |
|---|---|---|
| FS | Flexural Strength | MPa |
| FM | Flexural Modulus | MPa |
| FT | Fracture Toughness | MPa.m^{1/2} |
| PSS | Polymerization shrinkage stress | MPa |
| PSS red Control | Polymerization shrinkage stress Reduction vs Control (Δ) | % |
| Control | In the sense of this invention it is CMX Liq. (for resins) or Ceram X Universal (for pastes) | - |
| Max PSS rate | Maximum Polymerization shrinkage stress Rate | % |
| Max PSS rate red Control | Maximum Polymerization shrinkage stress Rate Reduction vs Control (Δ) | % |
| ΔH | Enthalpy | J/g |
| Exo Peak Slope | Slope of the exothermic peak | W/g/min |
| G'/G" croPSS over | An interaction between G' and G", the maximum of tan d. | see |
| CS | Compressive strength | MPa |
| ISO 20'-VLC DoC | Depth of polymerisation (cure) according to ISO 4049:2019(E) | mm |

### Experimental Methods:

Flexural Strength and Flexural Modulus: Specimens for 3-point bending flexural test were prepared according to ISO 4049:2019 "Dentistry - Polymer-basedrestorative materials". Sample was filled into 25mm x 2mm x 2mm stainless steel mold, then covered with Mylar film and cured using Spectrum 800 halogen lamp (Dentsply Sirona) at intensity of 850 mW/cm² for 5 x 20 seconds uniformly across the entire length of the specimen. The set specimens were stored in deionized water at 37°C for 24 hours prior to the test. Flexural test was conducted using a Universal Testing Machine Model 4400R (Instron) with crosshead speed 0.75 mm/min under compressive loading mode. A minimum of six specimens were tested for each set of samples.

3-point Fracture Toughness: To measure fracture toughness, samples were filled into 25 mm x 5 mm x 2 mm stainless steel molds, then covered by Mylar film and cured using Spectrum 800 halogen lamp (Dentsply Sirona) at intensity of 850 mW/cm² from both sides. After storage in DI-water at 37°C for 24-hour, a notch (depth 2.4 mm, width 0.35 mm) was created in the center of the specimen using a diamond cutting disc and the exact notch depth was measured with a VHX-2000 digital microscope (Keyence). Fracture toughness specimens were then fractured on a Universal Testing Machine Model 4400R (Instron) under 3-point bending mode at crosshead speed of 0.5 mm/min. A minimum of six specimens were tested for each set of samples.

Polymerization shrinkage stress: Polymerization shrinkage stress was measured with a polymerization shrinkage stress measurement device, referred to as a tensometer, designed and fabricated at the Paffenbarger Research Center of the American Dental Association Foundation (ADAF). This device is based on the cantilever beam theory that bending force generated by a shrinking sample during polymerization causes the cantilever beam to deflect. Polished top and bottom quartz rods (6.0 mm in diameter) were silanated with two layers of 2 vol% γ-methacryloxypropyltrimethoxysilane/acetone solution. Dental resin or composite are injected into a cell between two glass rods with 6.0 mm in diameter and 2.0 mm in thickness. Sample was then light cured using QHL-75 lamp (Dentsply Sirona) for 60 see at room temperature. The development of polymerization shrinkage stress was monitored continuously for 60 min and the highest polymerization shrinkage stress value is recorded. A minimum of 3 specimens were tested for each set of samples.

Photo-DSC: Photopolymerization kinetics studies were conducted with a differential scanning photo-calorimeter (Q200, TA Instruments), equipped with a High Pressure Mercury light source (OmniCure S2000 Spot Curing System). All tests were conducted in duplicate under nitrogen atmosphere at 25°C. The light intensity and irradiation wavelength were controlled with build-in band-pass filter with approximate output wavelength ranging from 400 nm to 500 nm. Polymerization exotherms were measured as specimen underwent photopolymerization. A minimum of three specimens were tested for each set of samples.

Photo-Rheology: Real-time photo-rheology studies were conducted with a rotational shear type rheometer (Discovery Hybrid Rheometer, DHR-2, TA Instruments), equipped with a photo-curing bottom-plate accessary, and high pressure mercury vapor short arc lamp (OmniCure S2000 Spot Curing System), High Power Fiber Light Guide. The light intensity and irradiation wavelength were controlled with build-in band-pass filter with approximate output wavelength ranging from 400 nm to 500 nm. Specimen was tested using Ø8mm Al plate geometry and initial thickness of 1mm, using fast sampling oscillation mode at 1hz shear frequency and 0.075% shear strain. Real time developments of shear storage modulus (G'), shear loss modulus (G"), gap position were measured as specimen underwent photopolymerization, upon exposure to photons guided via photo-curing bottom-plate accessary. A minimum of three specimens were tested for each set of samples.

FT-IR & FT-NIR Spectroscopy: FT-IR (mid-IR) & FT-NIR (near-IR) spectrum of neat E2PA, and CMX Liq. activated resins comprising various concentrations of E2PA were studied using Thermo Electron's Nicolet-6700 FTIR Spectrometer, with Smart Orbit Diamond ATR module used for mid-IR & DRIFT (Diffuse Reflectance Infrared Fourier Transform) module for near-IR. Spectrum were collected using number of scans of 32 and resolution of 4.

## Claims

1. A dental composition comprising
a. at least one polymerizable monomer
b. at least one polymerization initiator
c. at least one polymerization shrinkage mediator.

2. The dental composition of claim 1, further comprising at least one filler.

3. The dental composition according to claims 1-2, wherein the at least one polymerization shrinkage mediator is a phenylacrylate.

4. The dental composition of claim 3, wherein the phenylacrylate is Ethyl-2-phenylacrylate.

5. The dental composition of any of the preceding claims, wherein the at least one initiator is at least one photo polymerization initiator.

6. The dental composition of any of the preceding claims, wherein the at least one filler is selected from the group comprising calcium alumo silicate glass, calcium alumo fluorosilicate glass, calcium alumo fluoroborosilicate glass, strontium alumo silicate glass, strontium alumo fluorosilicate glass, strontium alumo fluoroborosilicate glass, and barium alumo borosilicate glass or barium alumo silicate glass.

7. The dental composition of claims 5-6, wherein the at least one photo polymerization initiator is selected from the group comprising mono and diacylphosphine oxides, camphorquinone, camphorquinone/amine, camphorquinone/triphenylgermanium hydride / diphenyl iodonium hexafluorophosphate, dimethyl-DPI and DKSI/DPI.

8. The dental composition according to any of the preceding claims, wherein the dental composition comprises 0.1-2.0 wt% of the at least one polymerization shrinkage mediator.

9. The dental composition of any of the preceding claims, wherein the purity of the phenylacrylate is 99%.

10. The dental composition of claims 1-9 for production of a dental restoration material, a dental adhesive, a dental cement, a post and core material, an endo-crown material or a pontic in fixed partial dentures.

11. The dental composition of claims 1-9 for medical applications.

## Amended claims

### Amended claims in accordance with Rule 137(2) EPC.

1. A dental composition comprising
a. at least one polymerizable monomer
b. at least one photoinitiator and
c. Ethyl-2-phenylacrylate
wherein the at least one polymerizable monomer is an acrylate or a methacrylate, **characterized in that** the dental composition comprises 0.625 -1.00 wt% of Ethyl-2- phenylacrylate and
**in that** the purity of Ethyl-2-phenylacrylate is 99% and
**in that** the Ethyl-2-phenylacrylate comprises a hindered carbon-centered radical.

2. The dental composition of claim 1, wherein the at least one photoinitiator is selected from the group comprising mono and diacylphosphine oxides, camphorquinone, camphorquinone and amine, camphorquinone and triphenylgermanium hydride and diphenyliodonium hexafluorophosphate, dimethyl-diphenyleneiodonium, tertbutyl(dimethylsilyl)glyoxylate and diphenyleneiodonium.

3. The dental composition of claims 1-2, further comprising at least one filler.

4. A dental restoration material, a dental adhesive, a dental cement, a post and core material, an endo-crown material or a pontic in fixed partial dentures comprising the dental composition of claims 1-3.

5. The dental composition of claims 1-4 for use in medical applications.
